# EUROPEAN PATENT SPECIFICATION

(11) **EP 3 896 167 B1**
(45) Date of publication and mention of the grant of the patent: **14.10.2026**
(21) Application number: 20894525.3
(22) Date of filing: 20.11.2020
(51) Int. Cl.: C12P 7/64, A23L 33/115, C12N 1/14, C12R 1/645

(54) **SCHIZOCHYTRIUM AND APPLICATION THEREOF, AND SN-2 DHA-RICH MICROBIAL OIL, PREPARATION METHOD THEREFOR, AND APPLICATION THEREOF**
SCHIZOCHYTRIUM UND SEINE ANWENDUNG SOWIE SN-2 DHA-REICHES MIKROBIELLES ÖL, VERFAHREN ZU SEINER HERSTELLUNG UND SEINE VERWENDUNG
SCHIZOCHYTRIUM ET SON APPLICATION, ET HUILE MICROBIENNE RICHE EN DHA SN-2, SON PROCÉDÉ DE PRÉPARATION ET SON APPLICATION

(30) Priority: 26.11.2019 CN 201911175787
(43) Date of publication of application: 20.10.2021
(73) Proprietor: Qu, Hanpeng, Changsha, Hunan 410036 (CN)
(72) Inventor: LIANG, Yun, Changsha, Hunan 410208 (CN); CAO, Sheng, Changsha, Hunan 410208 (CN); WANG, Shenjian, Changsha, Hunan 410208 (CN)
(74) Representative: Vitina, Maruta
(86) International application number: PCT/CN2020/130385
(87) International publication number: WO 2021/104165

(56) References cited:
- WO-A1-2013/013210
- CN-A- 103 725 721
- CN-A- 104 011 218
- CN-A- 109 247 397
- CN-A- 109 247 397
- CN-A- 110 846 346
- IWASAKI YUGO ET AL: "Enzymatic Synthesis of Structured Lipids from Single Cell Oil of High Docosahexaenoic Acid Content", JAOCS, VOL. 76, NO. 5, 1 January 1999 (1999-01-01), pages 563 - 569, XP055948203, Retrieved from the Internet <URL:https://link.springer.com/content/pdf/10.1007/s11746-999-0005-7.pdf> [retrieved on 20220802]

## Description

### TECHNICAL FIELD

The present disclosure relates to microbial technology, and more particularly to a *Schizochytrium* strain and a use thereof, a microbial oil containing DHA at an Sn-2 position, and a preparation and a use thereof.

### BACKGROUND

Docosahexaenoic acid (DHA) is a primary structural fatty acid in the brain and eyes, accounting for 97% and 93% of all n-3 fatty acids in the brain and eyes, respectively. It has been reported that the triglycerides with the Sn-2 position DHA are easier to be absorbed by intestinal mucosa. Meanwhile, it has also been demonstrated that when the intake of those lipids with the Sn-2 position DHA will make DHA most enriched in the brain, whereas when people take in lipids with DHA at other positions, of the highest level of DHA will occur in the liver, which indicates that triglycerides with different structures will experience different fatty acid metabolism routes, in other words, the fatty acids at the Sn-2 position can be more effectively absorbed than those at the Sn-1 or Sn-3 position. As a primary lipase, pancreatic lipase attaches to a water-oil interface to hydrolyze dietary fat molecules. Meanwhile, pancreatic lipase is specific to the hydrolysis of ester bonds at the Sn-1 and Sn-3 positions. As a consequence, after digested by pancreatic lipase, the triglyceride structure is converted into free fatty acids (from Sn-1 and Sn-3 positions) and a monoglyceride (formed by the glycerol skeleton and the Sn-2 fatty acid).The free fatty acids have difficulty in penetrating into bile salt micelle to be absorbed, and thus are prone to combining with calcium and magnesium ions in the intestine to form insoluble soap salts to be wasted, whereas the monoglyceride formed from the fatty acid at the Sn-2 position can easily penetrate into the bile salt micelle to be absorbed. Therefore, the absorption rate of fatty acids at the Sn-2 position in human body is higher than that of the fatty acids at the Sn-1 and Sn-3 positions.

As consumers become more aware of the health and function of DHA, microbial oils, as primary resources of DHA, have been largely adopted in infant food and nutraceuticals, and their nutritional benefits are increasingly recognized by public. Consequently, more and more attention has been paid to the absorption rate of DHA in microbial oils. More than 90% of the fatty acids in the microbial oil exist in the form of triglyceride, and the existing DHA-containing microbial oils are mainly produced by fermentation using *Ukenella, Schizochytrium, Thraustochytrium, Cryptodinium,* and yeast. However, the incorporation rate of DHA at Sn-2 position of the glycerol skeleton is far lower than that at the Sn-1 and Sn-3 positions, and a large amount of Sn-1 and Sn-3 DHA is converted into soap salts to be wasted, attenuating the benefits of the microbial oils.

Yugo Iwasaki et al. (Enzymatic Synthesis of Structured Lipids from Single Cell Oil of High Docosahexaenoic Acid Content, JAOCS, Vol. 76, no. 5, 1 January 1999 (1999-01-01), pages 563-569, XP055948203) reported enzymatic synthesis of structured lipids from Schizochytrium sp. SR21.

Chinese Patent Publication No. 110846346A, the priority document of the present application, discloses preparing a microbial oil comprising inoculating Schizochytrium strain into a fermentation medium and fermenting, where the Schizochytrium strain has deposition number of GDMCC No. 60733.

Chinese Patent Publication No. 104011218A discloses microbial oils that are enriched in their content of polyunsaturated fatty acids, their esters, their acid salts, alcohols formed therefrom, and/or aldehydes formed therefrom; compositions containing the enriched microbial oils and methods of making and using the enriched microbial oils; isolated microorganisms as well as strains and mutants thereof, biomasses, microbial oils, compositions, and cultures; methods of producing the microbial oils, biomasses, and mutants; and methods of using the isolated microorganisms, biomasses, and microbial oils.

Chinese Patent Publication No109247397A discloses a structured oil rich in docosahexaenoic acid (DHA) at a Sn-2 position and a preparation method thereof. In the structured oil of this invention, the Sn-2 position of a glycerol backbone is linked to DNA in an amount of 25% or more based on the total fatty acids of the structured oil, and Sn-1,3 positions are linked to fatty acids.

### SUMMARY

The object of the present invention is to provide a *Schizochytrium* strain and use thereof, a microbial oil containing DHA at an Sn-2 position and preparation and use thereof to solve the problem that human body has a poor adsorption to DHA in the microbial oil since DHA on the triglyceride in the microbial oil is dominated by Sn-1 and Sn-3 DHA. The *Schizochytrium* strain provided herein is *Schizochytrium* sp, and the microbial oil provided herein is produced through the fermentation using *Schizochytrium* sp, in which Sn-2 fatty acids of the triglyceride include 23% or more by weight of DHA, effectively improving the absorption of DHA in human body.

Technical solutions of this application are described as follows.

In a first aspect, the present disclosure provides a method for producing the microbial oil mentioned above, comprising:
inoculating a *Schizochytrium* strain into a fermentation medium for fermentation to produce the microbial oil;
wherein the *Schizochytrium* strain has an accession number of GDMCC No. 60733.

In a second aspect, the present invention provides a *Schizochytrium* strain, where the *Schizochytrium* strain has an accession number of GDMCC No. 60733.

In a third aspect, the present invention provides a use of the *Schizochytrium* strain in the preparation of the above-mentioned microbial oil.

The *Schizochytrium* sp.-derived microbial oil provided herein is rich in DHA, and a weight percentage of DHA at the Sn-2 position of the triglyceride in total Sn-2 fatty acids is not less than 23%, which effectively facilitates the absorption and utilization of DHA in the microbial oil in human body.

The features and beneficial effects will be further described in detail below with reference to the embodiments.

### Deposit of microorganisms

The *Schizochytrium* strain used herein has been deposited in Guangdong Microbial Culture Collection Center (GDMCC, Guangdong Institute of Microbiology, 5th Floor, No. 59 Building, No. 100 Xianliezhong Road, Guangzhou, China, 510070) on August 8, 2019 with an accession number of GDMCC No. 60733.

### DETAILED DESCRIPTION OF EMBODIMENTS

It should be noted that endpoints and values within ranges disclosed herein are only exemplary, and are intended to include any values close to these values or ranges. Any possible combination of values within the numerical range to form one or more new ranges should be considered to be expressly disclosed in this embodiment.

In the present embodiment, the total DHA is a total amount of DHA in the microbial oil, and can be measured according the method of GB 26400-2011. The contents of other fatty acids are measured according to the method of GB 5009.168-2016. Triglyceride DHA means that DHA is linked to the glycerol backbone through ester bonds.

In a first aspect, the present embodiment provides a method for producing the microbial oil. A *Schizochytrium* strain is inoculated into a fermentation medium for fermentation, where an accession number of the *Schizochytrium* strain is GDMCC No.60733.

The strain for preparing the microbial oil can be obtained by a conventional method in the art.

The *Schizochytrium* strain provided herein is obtained by mutagenesis.

The mutagenesis is performed according to a conventional method, such as physical mutagenesis (ultraviolet mutagenesis, atmospheric room temperature plasma (ARTP) mutagenesis) and chemical mutagenesis.

In some embodiments, the mutagenesis is performed by ARTP mutagenesis.

The ARTP mutagenesis is carried out in a conventional mutagenesis system such as multifunctional mutagenesis system (MPMS) produced by Adhoc Interteck Co., Ltd. (Beijing, China).

The mutagenesis is performed according to conventional operations in the art. In an embodiment, the mutagenesis is performed at a plasma mutagenesis power of 80-120 W, a gas flow rate of 8-12 SLM (standard liter per minute) and a treatment distance of 1-3 mm.

The mutagenesis time is 5-60 s, preferably 15-30 s.

The mutagenesis is performed on a bacterial suspension with OD₆₀₀ of 0.6-0.8 or a bacterial concentration of 10⁶-10⁸ cfu/mL.

In some embodiments, the mutagenesis is performed such that a lethality of the *Schizochytrium strain* is 90-95%.

The starting strain is subjected to mutagenesis and multiple screenings to obtain a strain with high oil yield and high Sn-2 position DHA content.

It should be understood by those skilled in the art that the percentage of the triglyceride with DHA at the Sn-2 position is used as an indicator for the screening of a desired high yield strain.

The content of Sn-2 fatty acids is determined according to the method recited in GB/T 24984-2010/ISO 6800:1997 "Animal and Vegetable Fats and Oils-Determination of the Composition of Fatty Acids in the 2-position of the Triglyceride Molecules".

In order to obtain a strain with high stability, the screened strain can also be evaluated for genetic stability. It has been accepted in the verification of genetic stability in the modern breeding that if the strain obtained from the mutation breeding can still meet the expected requirements of biological characteristics after five passages, it is considered to have high stability.

Through the mutagenesis, screening and genetic stability evaluation mentioned above, a strain with high yield of the Sn-2 DHA is obtained.

The *Schizochytrium* strain of the present embodiment has been deposited in Guangdong Microbial Culture Collection Center (GDMCC, Guangdong Institute of Microbiology, 5^{th} Floor, No. 59 Building, No. 100 Xianliezhong Road, Guangzhou, 510070, China) on August 8, 2019 with an accession number of GDMCC No. 60733.

After the fermentation, the *Schizochytrium* strain provided herein can produce an Sn-2 DHA-rich microbial oil, and the fermentation method has no special requirements as long as it enables the proliferation of the *Schizochytrium* strain.

There are no special requirements for the fermentation by the *Schizochytrium* strain. In some embodiments, the fermentation is performed at pH 6-7.5 and 27-31°C for 4-8 days under a ventilation rate of 0.5-1.1 vvm.

When the *Schizochytrium* strain is inoculated in the form of a seed liquid, the inoculation amount can be selected in a wide range, such as 5-10% (*v*/*v*).

The fermentation medium used herein can be a medium commonly used in the art for the fermentation by the *Schizochytrium* strain. In an embodiment, the fermentation medium includes a carbon source, a nitrogen source, an inorganic salt, a trace element and a vitamin.

The carbon source is glucose, sucrose, or other substances that can provide a carbon source or a combination thereof, and the nitrogen source is sodium glutamate, yeast powder, yeast extract or other substances that can provide a nitrogen source or a combination thereof.

In some embodiments, the carbon source is selected from the group consisting of glucose, sucrose and a combination thereof.

In some embodiments, the nitrogen source is selected from the group consisting of sodium glutamate, yeast powder, yeast extract and a combination thereof.

In some embodiments, the inorganic salt is selected from the group consisting of calcium salt, phosphate, potassium salt, sodium salt, magnesium salt, ammonium salt and a combination thereof.

In some embodiments, the trace element is selected from the group consisting of nickel, copper, molybdenum, cobalt, zinc, iron, manganese and a combination thereof.

In some embodiments, the vitamin is selected from the group consisting of vitamin B₁, vitamin B₁₂, vitamin B₆, calcium pantothenate, biotin and a combination thereof.

The carbon source, nitrogen source and inorganic salt can be directly added to a medium. However, when a volume of the medium is small, it is difficult to directly add the trace element and the vitamin to the medium, and at this time, the trace element and the vitamin are often prepared into a mother liquor to be added.

Contents of each component in the trace-element mother liquor can be selected within a wide range. In some embodiments, the trace-element mother liquor contains 1-3 g/L of nickel sulfate, 1-3 g/L of copper sulfate, 0.02-0.08 g/L of sodium molybdate, 2-4 g/L of manganese chloride, 0.02-0.08 g/L of cobalt chloride, 2-4 g/L of zinc sulfate and 8-10 g/L of ferrous sulfate.

Contents of each component in a vitamin mother liquor can be selected within a wide range. In some embodiments, the vitamin mother liquor contains 9-11 g/L of vitamin B₁, 0.1-0.3 g/L of vitamin B₁₂, 2-4 g/L of calcium pantothenate and 0.005-0.01 g/L of biotin.

In some embodiments, the nitrogen source in the fermentation medium includes glutamate.

A concentration of the glutamate can be selected in a wide range. In some embodiments, the concentration of the glutamate in the fermentation medium is 5-15 g/L.

A concentration of the carbon source can be selected in a wide range. In some embodiments, the concentration of the carbon source in the fermentation medium is 5-70 g/L.

In an embodiment, the fermentation medium contains 5-70 g/L of the carbon source, 15-45 g/L of the nitrogen source, 5-40 g/L of the inorganic salt, 0.01-0.04 g/L of the trace element and 0.01-0.04 g/L of the vitamin, preferably 40-60 g/L of the carbon source, 20-35 g/L of the nitrogen source, 10-25 g/L of the inorganic salt, 0.015-0.035 g/L of the trace element and 0.01-0.03 g/L of the vitamin.

In an embodiment, the fermentation medium further includes 0.1-0.5 g/L of an anti-foaming agent.

During the fermentation process, the addition of the carbon source and the nitrogen source is continuously performed to adjust the carbon-to-nitrogen ratio. When approaching the fermentation end, the carbon source is not supplied into the medium any more so that the residual sugar is reduced to 0. In this way, an auxotrophic condition is formed by adjusting the carbon-to-nitrogen ratio to improve the oil production of the *Schizochytrium* strains.

In order to increase a yield of the fermentation product, in some embodiments, the method includes: activating the *Schizochytrium* strain by shake flask culture to obtain a seed liquid; and inoculating the seed liquid into a seed culture medium followed by transferring to a fermentation medium for the fermentation. In some embodiments, the method includes: inoculating the *Schizochytrium* strain into an activation medium for activation to obtain an activated *Schizochytrium* suspension; inoculating the activated *Schizochytrium* suspension into a seed culture medium for proliferation to obtain a seed liquid; and inoculating the seed liquid into the fermentation medium for the fermentation to produce the microbial oil.

The *Schizochytrium* strain provided herein is preserved in an ampoule or a glycerin tube.

In some embodiments, in the activating process, the *Schizochytrium* strains stored in a frozen glycerin tube are thawed and inoculated into an activation medium for activation.

The activation can be performed once or multiple times to prepare the activated *Schizochytrium* suspension.

The activation conditions can be selected in a wide range. In some embodiments, the activation is carried out at 27.5-28.5°C and 150-200 r/min for 48-72 hours.

Preferably, the activation medium includes a carbon source, a nitrogen source, an inorganic salt, a trace element and a vitamin. In an embodiment, the activation medium contains 30-50 g/L of the carbon source, 25-45 g/L of the nitrogen source, 25-40 g/L of the inorganic salt, 0.015-0.025 g/L of the trace element and 0.01-0.02 g/L of the vitamin.

In an embodiment, the activation is performed through steps of: thawing the *Schizochytrium* strain stored in a frozen glycerin tube followed by inoculation into a sterilized activation medium using a sterile pipette; and culturing the *Schizochytrium* strain at 27.5-28.5°C and 150-200 r/min for 48-72 hours. In an embodiment, the activation medium contains 30-50 g/L of a carbon source, 25-45 g/L of a nitrogen source, 25-40 g/L of an inorganic salt, 0.015-0.025 g/L of a trace element and 0.01-0.02 g/L of a vitamin.

Preferably, the strain in one tube is inoculated into 4-6 500 mL flasks each containing 200-300 mL of the activation medium.

In the expansion culture, the activated *Schizochytrium* suspension is inoculated into a seed culture medium for expansion to obtain a seed liquid.

Preferably, the expansion culture is carried out at 27-28.5°C under a ventilation rate of 0.5-0.8 vvm for 48-72 hours.

Preferably, the seed culture medium includes 25-60 g/L of a carbon source, 10-30 g/L of a nitrogen source, 15-35 g/L of an inorganic salt, 0.01-0.02 g/L of a trace element and 0.01-0.02 g/L of a vitamin.

Preferably, the seed culture medium further includes 0.1-0.5 g/L of an anti-foaming agent.

In an embodiment, the expansion culture is performed through steps of: inoculating the activated *Schizochytrium* suspension into a primary seed culture medium for primary expansion followed by inoculation into a secondary seed culture medium for secondary propagation to obtain the seed liquid.

Preferably, the primary expansion is carried out at 27-28.5°C under a ventilation rate of 0.5-0.8 vvm for 48-60 hours.

Preferably, the secondary propagation is carried out at 27-28.5°C under a ventilation rate of 0.5-0.8 vvm for 12-24 hours.

Preferably, the primary seed culture medium contains 25-35 g/L of the carbon source, 10-30 g/L of the nitrogen source, 15-35 g/L of the inorganic salt, 0.01-0.02 g/L of the trace element and 0.01-0.02 g/L of the vitamin.

Preferably, the secondary seed culture medium contains 40-60 g/L of the carbon source, 10-15 g/L of the nitrogen source, 15-20 g/L of the inorganic salt, 0.01-0.02 g/L of the trace element and 0.01-0.02 g/L of the vitamin.

Types of components of the activation medium and the seed culture medium are the same as those of the fermentation medium,

The present embodiment may further process the above-mentioned fermentation product to obtain a microbial oil. There are no special requirements for the processing method as long as the method can extract the microbial oil from the fermentation product. In order to improve the production of the microbial oil, the fermentation product is subjected to wall breaking and extraction.

In a second aspect, the present embodiment provides a *Schizochytrium* strain with an accession number of GDMCC No. 60733.

The method for obtaining the *Schizochytrium* strain is described in the second aspect, and will not be repeated here.

In a third aspect, the present embodiment provides a use of the *Schizochytrium* strain in the production of the above-mentioned microbial oil.

Preferably, the microbial oil includes a triglyceride, where Sn-2 fatty acids of the triglyceride include 23% or more by weight of DHA.

Preferably, a weight percentage of total DHA in the microbial oil is not less than 38%.

The present embodiment will be further described in detail below with reference to the embodiments.

In the embodiments, a content of DHA and a fatty acid composition in a microbial oil are detected according to GB26400-2011 and GB 5009.168-2016, respectively.

The absorption rate of DHA in human body is detected by an efficacy trial, where male and female subjects are required to take in the microbial oil produced by the method of the present embodiment and a control DHA oil, and then blood samples are collected to determine a content of the DHA and a content of the Sn-2 DHA in the blood to calculate the absorption rate of DHA.

The conventional *Schizochytrium* strain is provided by China Center of Industrial Culture Collection, and has an accession number of CICC 11091s.

The glucose, sucrose, yeast powder, sodium glutamate, yeast extract, sodium chloride, magnesium sulfate, calcium chloride, potassium dihydrogen phosphate, nickel sulfate, copper sulfate, sodium molybdate, cobalt chloride, zinc sulfate, ferrous sulfate, manganese chloride, vitamin B₁, vitamin B₁₂, vitamin B₆, calcium pantothenate, biotin, sodium bicarbonate, sodium sulfate, ammonium sulfate and potassium chloride are all commercially available.

In the embodiments, the trace-element mother liquor contains 2 g/L of nickel sulfate, 1.9 g/L of copper sulfate, 0.04 g/L of sodium molybdate, 2.8 g/L of manganese chloride, 0.04 g/L of cobalt chloride, 3.2 g/L of zinc sulfate and 9 g /L of ferrous sulfate; and
the vitamin mother liquor contains 10.3 g/L of vitamin B₁, 0.16 g/L of vitamin B₁₂, 3.2 g/L of calcium pantothenate and 0.008 g/L of biotin.

### Preparation Example

### Preparation of Schizochytrium strain (GDMCC No.60733)

A parent strain preserved in the ampoule was inoculated into an activation medium and activated at 28°C for 2 days, where the activation medium contained 40 g/L of glucose, 31 g/L of sodium glutamate, 19 g/L of sodium chloride, 5.8 g/L of yeast extract, 8 g/L of magnesium sulfate, 5.7 g/L of potassium dihydrogen phosphate, 1 g/L of trace element and 1 g/L of vitamin (the activation medium plate further contained 18 g/L of agar).

The activated *Schizochytrium* suspension was spread on an activation medium plate, and then subjected to ARTP mutagenesis in a multifunctional mutagenesis system (MPMS) produced by Adhoc Interteck Co., Ltd. (Beijing, China), where the ARTP mutagenesis was carried out at a plasma mutagenesis power of 100 W, a gas flow rate of 10 SLM and a treatment distance of 2 mm for 25 s; the *Schizochytrium* suspension used for the mutagenesis had an OD₆₀₀ of 0.6-0.8 or a concentration of 10⁶-10⁸ cfu/mL; and a lethality rate was 92.54%. Well-grown single colonies were selected for passage, and then inoculated into a shake flask containing the activation medium and cultured at 27°C for 4 days. A preliminary screening was performed to detect a content of DHA and a content of the Sn-2 position DHA in the culture to select high-yield strains. The high-yield strains obtained by the preliminary screening were subjected to secondary screening by culture in a shake flask at 27°C for 4 days to further select high-yield strains. The genetic stability of the high-yield strains obtained by the secondary screening was investigated. After 5 passages, the strain with stable genetic traits was used as the production strain and stored for long-term use.

After screening, the *Schizochytrium* strain GDMCC No. 60733 of the present embodiment was obtained, which had been deposited in Guangdong Microbial Culture Collection Center (GDMCC, Guangdong Institute of Microbiology, 5th Floor, No. 59 Building, No. 100 Xianliezhong Road, Guangzhou, 510070, China) on August 8, 2019.

### Example 1

### 30 L fermentation and production using the Schizochytrium strain GDMCC No. 60733

An activation medium used herein contained 40 g/L of glucose, 31 g/L of sodium glutamate, 5.8 g/L of yeast extract, 19 g/L of sodium chloride, 8 g/L of magnesium sulfate, 5.7 g/L of potassium dihydrogen phosphate, 1 g/L of trace element and 1 g/L of vitamin.

A seed culture medium used herein contained 30 g/L of glucose, 6.3 g/L of sodium glutamate, 8.3 g/L of yeast extract, 8.3 g/L yeast powder, 1.45 g/L of sodium chloride, 5.18 g/L of magnesium sulfate, 1.66 g/L of potassium dihydrogen phosphate, 0.25 g/L of calcium chloride, 0.25 g/L of sodium bicarbonate, 9.34 g/L of sodium sulfate, 1.04 g/L of ammonium sulfate, 0.83 g/L of potassium chloride, 1 g/L of trace element, 1 g/L of vitamin and 0.3 g/L of a defoamer.

A fermentation medium used herein contained 50 g/L of glucose, 15 g/L of sodium glutamate, 10.9 g/L of yeast extract, 2.6 g/L of sodium chloride, 5.8 g/L of magnesium sulfate, 2.4 g/L of potassium dihydrogen phosphate, 0.25 g/L of calcium chloride, 0.22 g/L of sodium bicarbonate, 3.62 g/L of sodium sulfate, 1.13 g/L of ammonium sulfate, 0.94 g/L of potassium chloride, 1.1 g/L of a trace-element mother liquor, 1.1 g/L of a vitamin mother liquor and 0.19 g/L of a defoamer.

The fermentation was performed as follows.
(1) An ordinary *Schizochytrium* strain and the *Schizochytrium* strain GDMCC No. 60733 were activated, respectively. Specifically, the *Schizochytrium* strain stored in each frozen glycerin tube was thawed and inoculated into four shake flasks containing 200 mL of the activation medium, and cultured on a shaker at 28°C and 180 r/min for 48 hours to obtain an activated *Schizochytrium* suspension.
(2) The activated *Schizochytrium* suspension obtained from step (1) was inoculated into a shake flask containing 200 mL of the activation medium at 3% (v/v), and cultured on a shaker at 28°C and 180 r/min for 72 hours for proliferation.
(3) 200 mL of the culture obtained from step (2) was inoculated into a seed tank containing 3 L of the seed culture medium, and cultured at 28°C, 0.03 MPa and 180 r/min under a ventilation rate of 0.6 vvm for 50 hours.
(4) All of the culture in the seed tank was inoculated into a fermentation tank containing 14 L of the fermentation medium, and cultured at pH 6.8 and 29°C at a rotation speed of 140 r/min for 5-6 days, where the ventilation rate and the tank pressure were controlled at 0.95 vvm and 0.03 MPa, respectively. During the fermentation, a sterile glucose solution (250 g/L) and a sterile sodium glutamate solution (250 g/L) were added in fed-batch to maintain the glutamate concentration at 5-8 g/L and the carbon source concentration at 10-23 g/L. After 96 hours of the fermentation, the supply of carbon source and glutamate was stopped to obtain a fermentation broth.
(5) 5 L of the fermentation broth obtained from step (4) was subjected to enzymatic wall disruption, and then centrifuged by a high speed centrifuge to separate a water phase, a solid phase, and an oil phase to obtain a microbial oil.
(6) The microbial oil obtained from step (5) was analyzed to obtain a content of DHA, fatty acid composition and a weight percentage of DHA at the Sn-2 position of the triglyceride, and the results were showed in Table 1.

**Table 1 Parameters of the microbial oils in Example 1**

| | Ordinary *Schizochytrium* strain | *Schizochytrium* strain GDMCC No. 60733 |
|---|---|---|
| DHA (C22:6), g/100g | 40.256 | 43.139 |
| Palmitic acid (C16:0), g/100g | 21.894 | 20.974 |
| Stearic acid (C18:0), g/100g | 1.542 | 1.301 |
| Oleic acid (C18:1), g/100g | 0.312 | 0.273 |
| Docosapentaenoic acid (C22:5), g/100g | 11.012 | 10.330 |
| Weight percentage of DHA at Sn-2 position of triglyceride, % | 21.97 | 43.24 |
| Weight percentage of DHA at Sn-1 and Sn-3 positions of triglyceride, % | 56.98 | 47.55 |
| Absorption rate of DHA, % | 44.17 | 61.8 |

### Example 2

### 100 L fermentation and production using Schizochytrium strain GDMCC No. 60733

An activation medium used herein contained 30 g/L of glucose, 20 g/L of sodium glutamate, 5 g/L of yeast extract, 15 g/L of sodium chloride, 6 g/L of magnesium sulfate, 4 g/L of potassium dihydrogen phosphate, 0.8 g/L of trace element and 0.75 g/L of vitamin.

A seed culture medium used herein contained 25 g/L of glucose, 5.5 g/L of sodium glutamate, 7 g/L of yeast extract, 7 g/L of yeast powder, 1.1 g/L of sodium chloride, 4 g/L of magnesium sulfate, 1.2 g/L of potassium dihydrogen phosphate, 0.15 g/L of calcium chloride, 0.15 g/L of sodium bicarbonate, 7 g/L of sodium sulfate, 0.8 g/L of ammonium sulfate, 0.6 g/L of potassium chloride, 0.8 g/L of trace element, 0.75 g/L of vitamin and 0.2 g/L of a defoamer.

A fermentation medium used herein contained 30 g/L of glucose, 10 g/L of sucrose, 12 g/L of sodium glutamate, 9 g/L of yeast extract, 2 g/L of sodium chloride, 4.5 g/L of magnesium sulfate, 2 g/L of potassium dihydrogen phosphate, 0.2 g/L of calcium chloride, 0.2 g/L of sodium bicarbonate, 2.8 g/L of sodium sulfate, 1 g/L of ammonium sulfate, 0.8 g/L of potassium chloride, 0.9 g/L of trace element, 0.9 g/L of vitamin and 0.15 g/L of a defoamer.

The specific steps were shown as follows.
(1) The ordinary *Schizochytrium* strain and the *Schizochytrium* strain GDMCC No. 60733 were activated, respectively. The *Schizochytrium* strain stored in each frozen glycerin tube was thawed and inoculated into five shake flasks each containing 200 mL of the activation medium, and cultured on a shaker at 28.5°C and 150 r/min for 72 hours to obtain an activated *Schizochytrium* suspension.
(2) Two shake flasks of the activated *Schizochytrium* suspension obtained from step (1) were inoculated into five shake flasks each containing 200 mL of the activation medium, and cultured on a shaker at 28°C and 150 r/min for 48 hours.
(3) 400 mL of the culture obtained from step (2) was inoculated into a seed tank containing 6 L of the seed culture medium, and cultured at 28°C and 150 r/min for 48 hours, where a ventilation rate and a tank pressure were controlled at 0.5 vvm and 0.03 MPa, respectively.
(4) All of the culture in the seed tank was inoculated into a fermentation tank containing 45 L of the fermentation medium, and cultured at 28-29°C and 90-120 r/min for 5-6 days, where a ventilation rate and a tank pressure were controlled at 0.5-0.8 vvm and 0.03 MPa, respectively. During the fermentation, a sterile glucose solution (250 g/L) and a sterile sodium glutamate solution (250 g/L) were added in fed-batch to maintain a concentration of glutamate at 8-12 g/L and a concentration of carbon source at 40-65 g/L. After 96 hours of the fermentation, the supply of carbon source and glutamate was stopped to obtain a fermentation broth.
(5) 10 L of the fermentation broth obtained from step (4) was subjected to enzymatic wall disruption, and then centrifuged by a high speed centrifuge to obtain a microbial oil.
(6) The microbial oil obtained from step (5) was analyzed to obtain a content of DHA, a fatty acid composition and a weight percentage of DHA at the Sn-2 position of triglyceride, and the results were showed in Table 2.

**Table 2 Parameters of the microbial oils in Example 2**

| | Ordinary *Schizochytrium* strain | *Schizochytrium* strain GDMCC No. 60733 |
|---|---|---|
| DHA (C22:6), g/100g | 42.689 | 46.786 |
| Palmitic acid (C16:0), g/100g | 23.13 | 21.05 |
| Stearic acid (C18:0), g/100g | 1.56 | 1.31 |
| Oleic acid (C18:1), g/100g | 0.314 | 0.271 |
| Docosapentaenoic acid (C22:5), g/100g | 11.012 | 10.301 |
| Weight percentage of DHA at Sn-2 position of triglyceride, % | 22.76 | 43.68 |
| Weight percentage of DHA at Sn-1 and Sn-3 positions of triglyceride, % | 57.37 | 46.68 |
| Absorption rate of DHA, % | 43.98 | 62.8 |

### Example 3

45 m³ industrial fermentation and production using *Schizochytrium* strain GDMCC No.

### 60733

A seed culture medium used herein contained 50 g/L of glucose, 35 g/L of sodium glutamate, 10 g/L of yeast extract, 22 g/L of sodium chloride, 10 g/L of magnesium sulfate, 8 g/L of potassium dihydrogen phosphate, 1.4 g/L of trace element and 1.5 g/L of vitamin.

A primary seed culture medium used herein contained 35 g/L of glucose, 8 g/L of sodium glutamate, 10 g/L of yeast extract, 10 g/L of yeast powder, 1.9 g/L of sodium chloride, 6 g/L of magnesium sulfate, 2.1 g/L of potassium dihydrogen phosphate, 0.4 g/L of calcium chloride, 0.4 g/L of sodium bicarbonate, 12 g/L of sodium sulfate, 1.3 g/L of ammonium sulfate, 1.1 g/L of potassium chloride, 1.2 g/L of trace element, 1.2 g/L vitamin and 0.4 g/L of a defoamer.

A secondary seed culture medium was: 50 g/L of glucose, 5.65 g/L of sodium glutamate, 5.65 g/L of yeast extract, 1.3 g/L of sodium chloride, 4.65 g/L of magnesium sulfate, 1.49 g/L of potassium dihydrogen phosphate, 0.22 g/L of calcium chloride, 0.22 g/L of sodium bicarbonate, 8.39 g/L of sodium sulfate, 0.93 g/L of ammonium sulfate, 0.74 g/L of potassium chloride, 0.93 g/L of trace element, 0.93 g/L of vitamin and 0.3 g/L of a defoamer.

A fermentation medium used herein contained 40 g/L of glucose, 20 g/L of sucrose, 20 g/L of sodium glutamate, 12 g/L of yeast extract, 3.2 g/L of sodium chloride, 7 g/L of magnesium sulfate, 3 g/L of potassium dihydrogen phosphate, 0.4 g/L of calcium chloride, 0.3 g/L of sodium bicarbonate, 4.5 g/L of sodium sulfate, 1.5 g/L of ammonium sulfate, 1.2 g/L of potassium chloride, 1.6 g/L of trace element and 1.6 g/L of vitamin.

The specific steps of the fermentation were shown as follows.
(1) The ordinary *Schizochytrium* strain and the *Schizochytrium* strain GDMCC No. 60733 were activated, respectively. The *Schizochytrium* strain stored in each frozen glycerin tube were thawed and inoculated into six shake flasks containing 200 mL of the activation medium, and cultured on a shaker at 28°C and 180 r/min for 60 hours to obtain an activated *Schizochytrium* suspension.
(2) Two shake flasks of the activated *Schizochytrium* suspension obtained from step (1) were inoculated into five flasks each containing 200 mL of the activation medium, and cultured on a shaker at 28±0.5°C and 180 r/min for 60 hours for proliferation.
(3) 500 L of the primary seed culture medium was sterilized and cooled to 40°C or lower, and then transferred to a primary seed tank. 1 L of the culture obtained from step (2) was inoculated into the primary seed tank, and cultured at pH 6.8, 28±0.5°C and 150 r/min for 55 hours.
(4) 6 m³ of the secondary seed culture medium was sterilized and cooled to 40°C or lower, and then transferred to a secondary seed tank. All of the culture in the primary seed tank was aseptically inoculated into the secondary seed tank, and cultured at pH 6.8, 28±0.5°C and 150 r/min for 18 hours.
(5) 22 m³ of the fermentation medium was sterilized and cooled to 40°C or lower, and then transferred to a fermentation tank. All of the culture in the secondary seed tank was inoculated into the fermentation tank, and cultured at pH 7.5, 28±0.5°C and 100 r/min for 5 days, where a ventilation rate was controlled at 1.0 vvm. During the fermentation, a sterile glucose solution (250 g/L) and a sterile sodium glutamate solution (250 g/L) were added in fed-batch to maintain a concentration of glutamate at 12-15 g/L and a concentration of carbon source at 55-70 g/L. After 96 hours of the fermentation, the supply of carbon source and glutamate was stopped to obtain a fermentation broth.
(6) The fermentation broth was subjected to enzymatic wall disruption, preheated to 85-90°C, and then centrifuged by a triple-phase centrifuge to obtain a microbial oil.
(7) The microbial oil obtained from step (6) was analyzed to obtain a content of DHA, a fatty acid composition of and a weight percentage of DHA at the Sn-2 position of triglyceride, and the results were showed in Table 3.

**Table 3 Parameters of the microbial oils in Example 3**

| | Ordinary *Schizochytrium* strain | *Schizochytrium* strain GDMCC No. 60733 |
|---|---|---|
| DHA (C22:6), g/100g | 45.768 | 50.664 |
| Palmitic acid (C16:0), g/100g | 22.913 | 20.859 |
| Stearic acid (C18:0), g/100g | 1.321 | 1.293 |
| Oleic acid (C18:1), g/100g | 0.314 | 0.277 |
| Docosapentaenoic acid (C22:5), g/100g | 12.435 | 10.366 |
| Weight percentage of DHA at Sn-2 position of triglyceride, % | 22.148 | 43.96 |
| Weight percentage of DHA at Sn-1 and Sn-3 positions of triglyceride, % | 54.56 | 46.33 |
| Absorption rate of DHA, % | 44.78 | 63.1 |

## Claims

1. A method for producing a microbial oil, comprising:
inoculating a *Schizochytrium* strain into a fermentation medium for fermentation to produce the microbial oil;
wherein the *Schizochytrium* strain is *Schizochytrium* sp. GDMCC No. 60733;
the fermentation is carried out at pH 6-7.5 and 27-31°C for 4-8 days under a ventilation rate of 0.45-1.1 vvm;
the fermentation medium comprises 5-70 g/L of a carbon source, 15-45 g/L of a nitrogen source, 5-40 g/L of an inorganic salt, 0.01-0.04 g/L of a trace element and 0.01-0.04 g/L of a vitamin;
the nitrogen source comprises glutamate; and a concentration of glutamate in the fermentation medium is 5-15 g/L.

2. The method according to claim 1, **characterized in that** the method comprises:
inoculating the *Schizochytrium* sp. GDMCC No. 60733 into an activation medium for activation to obtain an activated *Schizochytrium* suspension;
inoculating the activated *Schizochytrium* suspension into a seed culture medium for expansion culture to obtain a seed liquid; and
inoculating the seed liquid into the fermentation medium for the fermentation to produce the microbial oil;
wherein the activation is carried out at 27.5-28.5°C and 150-200 r/min for 48-72 hours;
the activation medium comprises 30-50 g/L of a carbon source, 25-45 g/L of a nitrogen source, 25-40 g/L of an inorganic salt, 0.015-0.025 g/L of a trace element and 0.01-0.02 g/L of a vitamin;
the expansion culture is carried out at 27-28.5°C under a ventilation rate of 0.5-0.8 vvm for 48-72 hours; and
the seed culture medium comprises 25-60 g/L of a carbon source, 10-30 g/L of a nitrogen source, 15-35 g/L of an inorganic salt, 0.01-0.02 g/L of a trace element and 0.01-0.02 g/L of a vitamin.

3. The method according to claim 1 or 2, **characterized in that** the carbon source in the fermentation medium, the carbon source in the activation medium and the carbon source in the seed culture medium are independently selected from the group consisting of glucose, sucrose and a combination thereof;
the nitrogen source in the fermentation medium, the nitrogen source in the activation medium and the nitrogen source in the seed culture medium are independently selected from the group consisting of sodium glutamate, yeast powder, yeast extract and a combination thereof;
the inorganic salt in the fermentation medium, the inorganic salt in the activation medium and the inorganic salt in the seed culture medium are independently selected from the group consisting of calcium salt, phosphate, potassium salt, sodium salt, magnesium salt, ammonium salt and a combination thereof;
the trace element in the fermentation medium, the trace element in the activation medium and the trace element in the seed culture medium are independently selected from the group consisting of nickel, copper, molybdenum, cobalt, zinc, iron, manganese and a combination thereof; and/or
the vitamin in the fermentation medium, the vitamin in the activation medium and the vitamin in the seed culture medium are independently selected from the group consisting of vitamin B₁, vitamin B₁₂, vitamin B₆, calcium pantothenate, biotin and a combination thereof.

4. A *Schizochytrium* strain, **characterized in that** the *Schizochytrium* strain has an accession number of GDMCC No. 60733.

5. The *Schizochytrium* strain of claim 4 for use in the production of a microbial oil, **characterized in that** the microbial oil comprises a triglyceride;
wherein Sn-2 fatty acids of the triglyceride comprise 23% or more by weight of DHA; and
a weight percentage of total DHA in the microbial oil is not less than 38%.

## Patentansprüche

1. Verfahren zur Herstellung eines mikrobiellen Öls, umfassend:
Beimpfen eines Fermentationsmediums mit einem *Schizochytrium*-Stamm zur Fermentation, um das mikrobielle Öl herzustellen;
wobei es sich bei dem *Schizochytrium*-Stamm um *Schizochytrium* sp. GDMCC Nr. 60733 handelt;
wobei die Fermentation bei einem pH-Wert von 6-7,5 und bei 27-31 °C für 4-8 Tage bei einer Belüftungsrate von 0,45-1,1 vvm durchgeführt wird;
wobei das Fermentationsmedium 5-70 g/L einer Kohlenstoffquelle, 15-45 g/L einer Stickstoffquelle, 5-40 g/L eines anorganischen Salzes, 0,01-0,04 g/L eines Spurenelements und 0,01-0,04 g/L eines Vitamins umfasst;
und wobei die Stickstoffquelle Glutamat umfasst und eine Konzentration von Glutamat in dem Fermentationsmedium 5-15 g/L beträgt.

2. Verfahren nach Anspruch 1, **dadurch gekennzeichnet, dass** das Verfahren Folgendes umfasst:
Beimpfen eines Aktivierungsmediums mit *Schizochytrium* sp. GDMCC Nr. 60733 zur Aktivierung, um eine aktivierte *Schizochytrium*-Suspension zu erhalten;
Beimpfen eines Impfkulturmediums mit der aktivierten *Schizochytrium*-Suspension zur Vermehrungskultur, um eine Impfkulturflüssigkeit zu erhalten; und
Beimpfen des Fermentationsmediums mit der Impfkulturflüssigkeit zur Fermentation, um das mikrobielle Öl herzustellen;
wobei die Aktivierung bei 27,5-28,5 °C und einer Drehzahl von 150-200 r/min für 48-72 Stunden durchgeführt wird;
wobei das Aktivierungsmedium 30-50 g/L einer Kohlenstoffquelle, 25-45 g/L einer Stickstoffquelle, 25-40 g/L eines anorganischen Salzes, 0,015-0,025 g/L eines Spurenelements und 0,01-0,02 g/L eines Vitamins umfasst;
wobei die Vermehrungskultur bei 27-28,5 °C bei einer Belüftungsrate von 0,5-0,8 vvm für 48-72 Stunden durchgeführt wird; und
wobei das Impfkulturmedium 25-60 g/L einer Kohlenstoffquelle, 10-30 g/L einer Stickstoffquelle, 15-35 g/L eines anorganischen Salzes, 0,01-0,02 g/L eines Spurenelements und 0,01-0,02 g/L eines Vitamins umfasst.

3. Verfahren nach Anspruch 1 oder 2, **dadurch gekennzeichnet, dass** die Kohlenstoffquelle in dem Fermentationsmedium, die Kohlenstoffquelle in dem Aktivierungsmedium und die Kohlenstoffquelle in dem Impfkulturmedium jeweils unabhängig voneinander aus der Gruppe ausgewählt sind, die aus Glucose, Saccharose und einer Kombination davon besteht;
die Stickstoffquelle in dem Fermentationsmedium, die Stickstoffquelle in dem Aktivierungsmedium und die Stickstoffquelle in dem Impfkulturmedium jeweils unabhängig voneinander aus der Gruppe ausgewählt sind, die aus Natriumglutamat, Hefepulver, Hefeextrakt und einer Kombination davon besteht;
das anorganische Salz in dem Fermentationsmedium, das anorganische Salz in dem Aktivierungsmedium und das anorganische Salz in dem Impfkulturmedium jeweils unabhängig voneinander aus der Gruppe ausgewählt sind, die aus Calciumsalz, Phosphat, Kaliumsalz, Natriumsalz, Magnesiumsalz, Ammoniumsalz und einer Kombination davon besteht;
das Spurenelement in dem Fermentationsmedium, das Spurenelement in dem Aktivierungsmedium und das Spurenelement in dem Impfkulturmedium jeweils unabhängig voneinander aus der Gruppe ausgewählt sind, die aus Nickel, Kupfer, Molybdän, Kobalt, Zink, Eisen, Mangan und einer Kombination davon besteht; und/oder
das Vitamin in dem Fermentationsmedium, das Vitamin in dem Aktivierungsmedium und das Vitamin in dem Impfkulturmedium jeweils unabhängig voneinander aus der Gruppe ausgewählt sind, die aus Vitamin B₁, Vitamin B₁₂, Vitamin B₆, Calciumpantothenat, Biotin und einer Kombination davon besteht.

4. *Schizochytrium*-Stamm, **dadurch gekennzeichnet, dass** der *Schizochytrium*-Stamm die Hinterlegungsnummer GDMCC Nr. 60733 aufweist.

5. *Schizochytrium*-Stamm nach Anspruch 4 zur Verwendung bei der Herstellung eines mikrobiellen Öls, **dadurch gekennzeichnet, dass** das mikrobielle Öl ein Triglycerid umfasst;
wobei der Gewichtsanteil von DHA, bezogen auf die Gesamtheit der Sn-2-Fettsäuren des Triglycerids, 23 % oder mehr beträgt; und
wobei der Gewichtsanteil von Gesamt-DHA in dem mikrobiellen Öl nicht weniger als 38 % beträgt.

## Revendications

1. Procédé de production d'une huile microbienne, comprenant :
inoculer une souche de *Schizochytrium* dans un milieu de fermentation pour une fermentation afin de produire l'huile microbienne ;
dans lequel la souche de *Schizochytrium* est *Schizochytrium* sp. GDMCC n° 60733 ;
la fermentation est effectuée à pH 6-7,5 et à 27-31 °C pendant 4-8 jours sous un taux d'aération de 0,45-1,1 vvm ;
le milieu de fermentation comprend 5-70 g/L d'une source de carbone, 15-45 g/L d'une source d'azote, 5-40 g/L d'un sel inorganique, 0,01-0,04 g/L d'un oligo-élément et 0,01-0,04 g/L d'une vitamine ;
la source d'azote comprend du glutamate ; et une concentration de glutamate dans le milieu de fermentation est de 5-15 g/L.

2. Procédé selon la revendication 1, **caractérisé en ce que** le procédé comprend :
inoculer le *Schizochytrium* sp. GDMCC n° 60733 dans un milieu d'activation pour une activation afin d'obtenir une suspension de *Schizochytrium* activée ;
inoculer la suspension de *Schizochytrium* activée dans un milieu de culture de semence pour une culture d'amplification afin d'obtenir un bouillon de semence ; et
inoculer le bouillon de semence dans le milieu de fermentation pour la fermentation afin de produire l'huile microbienne ;
dans lequel l'activation est effectuée à 27,5-28,5 °C et 150-200 tr/min pendant 48-72 heures ;
le milieu d'activation comprend 30-50 g/L d'une source de carbone, 25-45 g/L d'une source d'azote, 25-40 g/L d'un sel inorganique, 0,015-0,025 g/L d'un oligo-élément et 0,01-0,02 g/L d'une vitamine ;
la culture d'amplification est effectuée à 27-28,5 °C sous un taux d'aération de 0,5-0,8 vvm pendant 48-72 heures ; et
le milieu de culture de semence comprend 25-60 g/L d'une source de carbone, 10-30 g/L d'une source d'azote, 15-35 g/L d'un sel inorganique, 0,01-0,02 g/L d'un oligo-élément et 0,01-0,02 g/L d'une vitamine.

3. Procédé selon la revendication 1 ou 2, **caractérisé en ce que** la source de carbone dans le milieu de fermentation, la source de carbone dans le milieu d'activation et la source de carbone dans le milieu de culture de semence sont choisies indépendamment dans le groupe constitué par le glucose, le saccharose et une combinaison de ceux-ci ;
la source d'azote dans le milieu de fermentation, la source d'azote dans le milieu d'activation et la source d'azote dans le milieu de culture de semence sont choisies indépendamment dans le groupe constitué par le glutamate de sodium, la poudre de levure, l'extrait de levure et une combinaison de ceux-ci ;
le sel inorganique dans le milieu de fermentation, le sel inorganique dans le milieu d'activation et le sel inorganique dans le milieu de culture de semence sont choisis indépendamment dans le groupe constitué par un sel de calcium, un phosphate, un sel de potassium, un sel de sodium, un sel de magnésium, un sel d'ammonium et une combinaison de ceux-ci ;
l'oligo-élément dans le milieu de fermentation, l'oligo-élément dans le milieu d'activation et l'oligo-élément dans le milieu de culture de semence sont choisis indépendamment dans le groupe constitué par le nickel, le cuivre, le molybdène, le cobalt, le zinc, le fer, le manganèse et une combinaison de ceux-ci ; et/ou
la vitamine dans le milieu de fermentation, la vitamine dans le milieu d'activation et la vitamine dans le milieu de culture de semence sont choisies indépendamment dans le groupe constitué par la vitamine B₁, la vitamine B₁₂, la vitamine B₆, le pantothénate de calcium, la biotine et une combinaison de ceux-ci.

4. Souche de *Schizochytrium*, **caractérisée en ce que** la souche de *Schizochytrium* a un numéro d'enregistrement GDMCC n° 60733.

5. Souche de *Schizochytrium* selon la revendication 4 pour une utilisation dans la production d'une huile microbienne, **caractérisée en ce que** l'huile microbienne comprend un triglycéride ;
dans laquelle les acides gras en position Sn-2 du triglycéride comprennent 23 % ou plus en poids de DHA ; et
un pourcentage en poids de DHA total dans l'huile microbienne n'est pas inférieur à 38 %.
